# EUROPEAN PATENT APPLICATION

(11) **EP 4 570 843 A1**
(43) Date of publication of application: **18.06.2025**
(21) Application number: 22968137.4
(22) Date of filing: 14.12.2022
(51) Int. Cl.: C08G 18/75, C07C 263/10, C07C 265/14

(54) **ISOPHORONE DIISOCYANATE COMPOSITION AND BLOCKED PRODUCT THEREOF**

(71) Applicant: Wanhua Chemical Group Co., Ltd., Yantai, Shandong 264006 (CN)
(72) Inventor: HUA, Weiqi, Chongqing Street, YEDA, Yantai, Shandong 264006 (CN); YU, Yong, Chongqing Street, YEDA, Yantai, Shandong 264006 (CN); LI, Jianfeng, Chongqing Street, YEDA, Yantai, Shandong 264006 (CN); SUN, Shuchang, Chongqing Street, YEDA, Yantai, Shandong 264006 (CN); CUI, Xuelei, Chongqing Street, YEDA, Yantai, Shandong 264006 (CN); WANG, Jingxu, Chongqing Street, YEDA, Yantai, Shandong 264006 (CN); SHANG, Yonghua, Chongqing Street, YEDA, Yantai, Shandong 264006 (CN); LIU, Degang, Chongqing Street, YEDA, Yantai, Shandong 264006 (CN); FAN, Weijing, Chongqing Street, YEDA, Yantai, Shandong 264006 (CN); LI, Yuan, Chongqing Street, YEDA, Yantai, Shandong 264006 (CN)
(74) Representative: Potter Clarkson
(86) International application number: PCT/CN2022/138925
(87) International publication number: WO 2024/124426

(57) **Abstract**

The present application relates to an isophorone diisocyanate composition and the use thereof. The content of methylated isophorone diisocyanate (Me-IPDI) in the isophorone diisocyanate composition is 0.002%-0.200%. A blocked isocyanate obtained by further blocking the IPDI provided in present application has a lower deblocking temperature and deblocking stability.

## Description

### FIELD OF THE INVENTION

Embodiments of the present application relate to an isophorone diisocyanate composition and blocked product thereof.

### BACKGROUND OF THE INVENTION

Isophorone diisocyanate (abbreviated as IPDI), a colorless or light yellow liquid at room temperature, is an aliphatic isocyanate and a cycloaliphatic isocyanate. It has a lower reactivity and vapor pressure than aromatic isocyanates, and is relatively less toxic than aromatic isocyanates. Due to the absence of benzene rings in its structure, it has good weathering resistance and can be used to prepare high-grade polyurethane materials with light stability, weathering resistance, and excellent mechanical properties, such as polyurethane elastomers, waterborne polyurethane dispersions, polyurethane UV resins, etc. IPDI can also self-polymerize to generate polyisocyanates with multiple functional groups, and the surface of the coating prepared with it dries quickly, making it have excellent application in automotive repair paints. In the aforesaid applications, the requirements for various compositions and impurity contents in IPDI monomers are relatively strict.

The blocked isocyanate is generated by reacting isocyanates with blocking agents. The chemical bond formed between the blocking agent and isocyanate group (NCO) is relatively weak, and under certain conditions, it can be deblocked and free NCO can be released. Therefore, the blocked isocyanate is widely used in single-component polyurethane coatings and adhesives, particularly suitable for automotive coatings and coil coatings. There are many types of blocking agents that can be used for isocyanate groups. Currently, the commonly used blocking agents include phenolic compounds, alcohol compounds, oxime compounds, *β*-dicarbonyl compounds, pyrazole compounds, amide compounds, etc. Among them, oxime compounds are widely used as isocyanate blocking agents due to their relatively low deblocking temperature. Although the deblocking temperature of the blocked isocyanate blocked by oxime compounds is relatively low, it is still around 140 °C. During the deblocking process, prolonged heating can easily cause the situation that isocyanates will turn yellow in color, thereby affecting the application of the product in transparent paints and light-colored paints. For example, patent documents US5504178, US5631339, and EP0829500 all disclose the use of partially hydrazide structured substances as stabilizers to improve the problem of heat-yellowing that occurs during product application. Although the use of the technology of the above patents can improve the color stability of blocked isocyanate products during storage to a certain extent, there are very few solutions to the problem of color stability during the deblocking process.

### SUMMARY OF THE INVENTION

The following is a summary of the subject matter described in detail herein. This summary is not intended to limit the scope of protection of the claims.

Embodiments of the present application provide an isophorone diisocyanate composition, which reduces the temperature during the deblocking process from the perspective of the composition of the isophorone diisocyanate composition itself, thereby solving the problem that the blocked isophorone diisocyanate products turn yellow in color during the deblocking process.

An isophorone diisocyanate (IPDI) composition, wherein the mass content of methylated isophorone diisocyanate (Me-IPDI) is in a range from 0.002% to 0.200%, preferably in a range from 0.005% to 0.075%, and more preferably in a range from 0.010% to 0.060%.

The structural formula of Me-IPDI is: 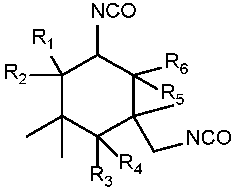 wherein at least one of R₁, R₂, R₃, R₄, R₅, and R₆ is CH₃, and the others are H, preferably at least one of R₁, R₃, and R₅ is CH₃;

More preferably, the structure of Me-IPDI is one or more selected from the group consisting of the following structures:

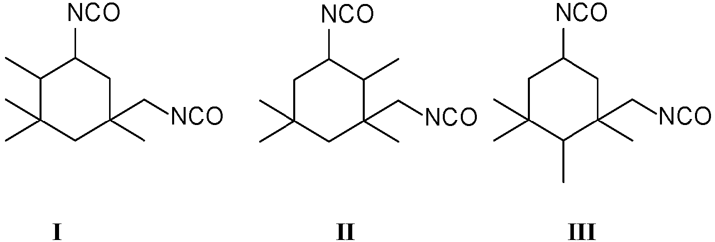

There is no specific requirement for the preparation method of the isophorone diisocyanate composition in the present application. The isophorone diisocyanate composition with such constituents can be obtained through any feasible method in related technologies.

Patent CN109761855A mentioned a whole industry chain preparation method for isophorone diisocyanate, and the preparation process of IPDI comprises: (1) in the presence of catalyst, reacting isophorone with hydrogen cyanide to obtain isophorone nitrile; (2) reacting the isophorone nitrile obtained in step (1), ammonia, and hydrogen in the presence of catalyst to obtain isophorone diamine; and (3) performing phosgenation reaction on isophorone diamine to obtain isophorone diisocyanate. According to the technology provided by this patent, three key raw materials (isophorone (IP), isophorone nitrile (IPN), and isophorone diamine (IPDA)), as well as three key reaction steps (cyanidation, hydrogenation, and phosgenation) were used respectively in the production process of IPDI.

The generation of Me-IPDA can be controlled by controlling the chloride content in the catalyst used during the preparation of IPDA from IPN. During the production, storage, or use of catalysts, chloride ions are introduced more or less, which react with metals inside the catalyst or during use to form metal chlorides. These types of chlorides catalyze the alkylation side reaction in the preparation of IPDA from IPN, thereby leading to the generation of Me-IPDA.

The applicant of the present application has found through research that when the chlorine content in the hydrogenation catalyst is controlled at 0.0002-0.0200%, preferably 0.0010-0.0150%, and more preferably 0.0020-0.0200%, the content of Me-IPDA in the IPDA obtained from the preparation process from IPN to IPDA can be controlled at 0.002%-0.200%, preferably 0.005%-0.075%, and more preferably 0.010%-0.060%. Further phosgenation can obtain IPDI with a Me-IPDI content of 0.002%-0.200%, preferably 0.005%-0.075%, and more preferably 0.010%-0.060%.

There may be other methods for controlling Me-IPDA, which will not be listed one by one in the present application. The previously mentioned plurality of methods can be used in combination or separately, and the method of obtaining the IPDI composition is not limited in the present application.

The Me-IPDI content in the isophorone diisocyanate composition in the present application can be analyzed by injecting samples using gas chromatography method. There is no specific requirement in the present application. For example, in some specific examples, the method used is as follows: the sample is dissolved using a solvent (preferably dichloromethane) and then analyzed by injecting samples using gas chromatography method, detected by hydrogen ion flame detector (FID), and quantitatively calculated by area normalization method.

The isophorone diisocyanate composition in the present application can be further prepared into blocked products. The blocked product prepared by the IPDI composition provided in the present application has the characteristics of lower deblocking temperature, stable product after deblocking, and the like.

Embodiments of the present application also provide a method for preparing a blocked isophorone diisocyanate which is storage-stable. The blocked isophorone diisocyanate is obtained by contacting the IPDI composition provided in the present application with a blocking agent, and performing reaction.

According to the research conducted by the applicant, it is found that the blocked isocyanate composition prepared using the IPDI composition containing a certain amount of Me-IPDI provided in the present application has lower deblocking temperature and obviously lower Chromatic Number (APHA) after deblocking.

It is speculated that the Me-IPDI contained in the IPDI composition has one and/or more additional methyl groups on the six-membered ring compared to conventional IPDI, resulting in a low bond energy of the blocked structure after forming the blocked product, therefore, the temperature required for deblocking is relatively lower. In addition, according to the knowledge of those skilled in the art, isocyanate products are prone to polymerization under the circumstances of long-term high temperature heating, which can further lead to an increase in Chromatic Number. However, the blocked product formed by using the IPDI composition provided in the present application has lower deblocking temperature, thereby the Chromatic Number increase during the deblocking process is relatively smaller.

In theory, the blocked product prepared from IPDI compositions with higher Me-IPDI content also has lower deblocking temperature, but during actual testing process, it was found that the purity of the IPDI composition decreased and its thermal stability also decreased when the Me-IPDI content was higher, therefore, the Chromatic Number of the product during the deblocking process also has a significant increase. Therefore, the present application limits the Me-IPDI content in the IPDI composition to 0.002%-0.200%, preferably 0.005%-0.075%, and more preferably 0.010%-0.060%.

According to the preparation method provided in the present application, in some examples, the blocking agent is one or more selected from the group consisting of oxime compounds, alcohol compounds, lactam compounds, pyrazole compounds, and *β*-dicarbonyl compounds.

According to the preparation method provided in the present application, in the preferred embodiments, the oxime compound is one or more selected from the group consisting of butanone oxime, acetone oxime, formaldehyde oxime, acetaldehyde oxime, and cyclohexanone oxime, and more preferably butanone oxime.

In some examples, the molar amount of the oxime compound accounts for 80 mol% or more of the total molar amount of the blocking agent (e.g., 85 mol%, 88 mol%, 90 mol%, 95 mol%, 100 mol%).

According to the preparation method provided in the present application, in some examples, the blocking agent further comprises one or more of alcohol compounds, lactam compounds, pyrazole compounds, and *β*-dicarbonyl compounds. Preferably, the blocking agent further comprises ε-caprolactam and/or 3,5-dimethylpyrazole.

In some examples, the alcohol compounds may be one or more selected from the group consisting of methanol, ethanol, 2-propanol, n-butanol, sec-butanol, 2-ethyl-1-hexanol, 2-methoxyethanol, 2-ethoxyethanol, and 2-butoxyethanol. The lactam compounds may be one or more selected from the group consisting of ε-caprolactam, *δ*-valerolactam, and *γ*-butyrolactam. The pyrazole compounds may be one or more selected from the group consisting of pyrazole, 3-methylpyrazole, and 3,5-dimethylpyrazole. The *β*-dicarbonyl compounds may be one or more selected from the group consisting of dimethyl malonate, diethyl malonate, ethyl acetoacetate, methyl acetoacetate, di-n-propyl malonate, diisopropyl malonate, di-n-butyl malonate, and diisobutyl malonate.

According to the preparation method provided in the present application, preferably, the ratio of the molar amount of NCO in the IPDI composition to the molar amount of the blocking agent is (0.9-1):1 (e.g., 0.95:1, 0.97:1, 0.99:1, 1:1).

According to the preparation method provided in the present application, the process conditions of the reaction include: the reaction temperature is 30-120 °C (e.g., 40 °C, 60 °C, 80 °C, 100 °C, 110 °C). There is no specific regulation on the reaction time in this step. For example, under stirring conditions, the reaction is carried out until the characteristic absorption peak of NCO could not be detected by infrared spectroscopy, resulting in a blocked isocyanate composition.

According to the preparation method provided in the present application, in some examples, a solvent that is reactive inert towards NCO groups is added into the reaction system.

In some examples, the solvent is one or more selected from the group consisting of ethyl acetate, butyl acetate, 1-methoxy-2-propyl acetate, 3-methoxy-n-butyl acetate, acetone, butanone, 4-methyl-2-pentanone, cyclohexanone, toluene, xylene, and S100 solvent oil, preferably, the solvent is one or more selected from the group consisting of S100 solvent oil, n-butyl acetate, and 1-methoxy-2-propyl acetate. The amount of solvent added mentioned here, for example, should be sufficient to completely dissolve the polyisocyanate and the blocking agent in the reaction system.

According to the preparation method provided in the present application, inert gas protection can be used during the reaction process to further reduce the chrominance of the blocked polyisocyanate composition product. The inert gas includes but is not limited to one or more of N₂, CO₂, CO, He, and Ar, preferably N₂.

Compared with the related technology, the beneficial effects of the blocked product prepared from the isophorone diisocyanate composition provided in the embodiments of the present application lie in the following aspects:
1. Compared with the blocked product prepared by conventional IPDI, the blocked product prepared using the IPDI composition provided in the embodiments of the present application has lower deblocking temperature; and
2. The increase degree of the Chromatic Number of the blocked product prepared using the IPDI composition provided in the embodiments of the present application is lower than that of the blocked product prepared using conventional IPDI during the deblocking process.

Other aspects can be understood after reading and understanding detailed description.

### DETAILED DESCRIPTION OF THE INVENTION

The technical solution of the present application will be further illustrated by the following specific embodiments. Those skilled in the art should understand that the examples are merely intended to assist in understanding the present application, and should not be considered as specific limitations on the present application.

In the following examples and comparative examples, the sources of main raw materials are as follows:
Butanone oxime, purchased from Hubei Xianlin Chemical Co., Ltd;
S100 solvent oil, purchased from ExxonMobil Chemical; and

The Chromatic Number of the blocked isocyanate composition was tested using a BYK colorimeter.

The content of the methylated isophorone diisocyanate in the isophorone diisocyanate composition can be analyzed by injecting samples using gas chromatography method. The method is as follows: the sample is dissolved using dichloromethane and then analyzed by injecting samples using gas chromatography method, detected by hydrogen ion flame detector (FID), and quantitatively calculated by area normalization method.

The chromatography conditions are as follows:
Carrier gas: Purified and dried high-purity nitrogen (purity ≥ 99.999%);
Combustion gas: Hydrogen (purity ≥ 99.999%) with a flow rate of 40 mL/min;
Combustion-supporting gas: Purified and dried air with a flow rate of 400 mL/min;
Make-up gas: Nitrogen with a flow rate of 30 mL/min;
Column flow rate: 1.06 mL/min;
Split ratio: 30:1;
Column temperature (programmed heating): Holding at 140 °C for 0 minutes, heating up to 220 °C at a rate of 10 °C/min, holding for 1 minute, heating up to 260 °C at a rate of 5 °C/min, holding for 0 minute, heating up to 280 °C at a rate of 10 °C/min, and holding for 1 minute;
Injection port temperature: 270 °C;
Detector temperature: 290 °C;
Sample size: 0.2 µL.

The analysis of the Chromatic Number of the blocked isocyanate composition adopts the method mentioned in China national standard GB/T605-2006.

The analysis of turbidity index of the blocked isocyanate composition adopts the method mentioned in China national standard GB/T605-2006.

### Example 1 - Preparation of IPDI composition with different Me-IPDI contents

### Step A: Preparation of isophorone diisocyanate composition with Me-IPDI content less than 0.002%

Through the method provided in patent CN109761855A, the synthesis of IPDI was carried out according to the following steps:
(1) isophorone was fed into a pre-heater at a rate of 200 kg/h and pre-heated to a reaction temperature of 120 °C, then, isophorone, HCN and an alkaline catalyst (sodium methoxide) were sent separately into the reactor disclosed in Example 1 of CN103301799B under the operating conditions disclosed in Example 1 of CN103301799B in a molar ratio of 2:1:0.003 for reaction. The reaction pressure was 1MPa absolute pressure, and isophorone nitrile (3-cyano-3,5,5-trimethylcyclohexanone, abbreviated as IPN) was obtained after reacting for 25 minutes;
(2) the isophorone nitrile obtained above was reacted with ammonia and hydrogen in the presence of catalyst, specifically as follows:
   a) the isophorone nitrile obtained in step (1) was reacted with ammonia in a tubular reactor at a temperature of 60 °C and an absolute pressure of 15MPa, with a molar ratio of ammonia to isophorone nitrile being 50:1, and 3-cyano-3,5,5-trimethylcycloheximide was obtained;
   b) in the presence of hydrogenation catalyst Raney cobalt (the catalyst has a space velocity of 1.5 g 3-cyano-3,5,5-trimethylcyclohexanone/(mL catalyst•hour)), 3-cyano-3,5,5-trimethylcycloheximide obtained in step a), hydrogen, and NH₃ were mixed and reacted in an ethanolic solution of 3% KOH. The reaction was carried out at a temperature of 80 °C and an absolute pressure of 18MPa to obtain a product containing 3-aminomethyl-3,5,5-trimethylcyclohexylamine (abbreviated as IPDA) and 3-cyano-3,5,5-trimethylcyclohexylamine;
      in step b), a mass ratio of the ethanolic solution of KOH to the added isophorone nitrile was 1:600, a molar ratio of NH₃ to isophorone nitrile was 50:1, and a molar ratio of hydrogen to isophorone nitrile was 80:1;
   c) in the presence of hydrogenation catalyst Raney cobalt (the catalyst has a space velocity of 1.8g 3-cyano-3,5,5-trimethylcyclohexanone/(mL catalyst•hour)), the product containing 3-aminomethyl-3,5,5-trimethylcyclohexylamine and 3-cyano-3,5,5-trimethylcyclohexylamine obtained in step b), hydrogen and NH₃ were mixed and reacted in an ethanolic solution of 3% acetic acid. The reaction was carried out at a temperature of 120 °C and an absolute pressure of 18MPa to convert 3-cyano-3,5,5-trimethylcyclohexylamine to 3-aminomethyl-3,5,5-trimethylcyclohexylamine.
      In step c), a mass ratio of the ethanolic solution of acetic acid to IPN obtained in step 1) was 1:500, a molar ratio of hydrogen to IPN obtained in step 1) was 30:1, and a molar ratio of ammonia to IPN obtained in step a) was 50:1;
      The chlorine content of the catalyst Reney cobalt used in each step of step (2) was 0.0001%;
(3) The obtained IPDA was gasified and heated to 355 °C by using the heater mentioned in Example 1 of Chinese patent CN105214568A, then, under nitrogen protection, IPDA and gaseous phosgene which was heated to 355 °C were continuously added into the reactor through their respective feed pipes for reaction. The reaction was carried out at an absolute pressure of 0.05MPa and a temperature of 360 °C; wherein the feed rate of IPDA was 800 Kg/h, and the feed rate of phosgene was 3000 Kg/h. The mixed gas after the reaction was rapidly cooled to 100 °C via a gas jet-absorption device with o-dichlorobenzene solution to obtain a photochemical solution containing the product IPDI. Excess phosgene was removed at 180 °C and an absolute pressure of 0.1MPa to obtain crude IPDI product without phosgene. Subsequently, the crude product was distilled through a distillation tower to obtain IPDI product which was taken at 0.5KPa and a distillation range of 150-160 °C, with a yield of 95% and a purity of 99.85%, wherein the Me-IPDI content (the sum of the contents of formulas I, II, and III, the same below) was 0.0003% (sample 1).

### Step B: Preparation of isophorone diisocyanate (IPDI) composition with Me-IPDI

### content greater than 0.200%

The difference from step A lies in: the chlorine content of the catalyst Raney cobalt used in each step of step (2) was 0.025%, and the solvent used in each step was methanol. The Me-IPDI content in the obtained IPDI was 0.32% (sample 2).

### Step C: Preparation of IPDI compositions with different Me-IPDI contents

The obtained sample 1 and sample 2 were mixed in a certain proportion to obtain IPDI compositions with different Me-IPDI contents, named as Mixed samples 1 to 14, wherein the Me-IPDI contents of Mixed samples 2 to 13 meet the requirements of the IPDI composition in the present application. The specific Me-IPDI contents in the mixed IPDI compositions are shown in Table 1 below.

**Table 1. IPDI Composition Samples and Me-IPDI Contents**

| | Me-IPDI content after mixing / % |
|---|---|
| Mixed sample 1 | 0.0009 |
| Mixed sample 2 | 0.0029 |
| Mixed sample 3 | 0.0041 |
| Mixed sample 4 | 0.0070 |
| Mixed sample 5 | 0.0089 |
| Mixed sample 6 | 0.0201 |
| Mixed sample 7 | 0.0300 |
| Mixed sample 8 | 0.0399 |
| Mixed sample 9 | 0.0502 |
| Mixed sample 10 | 0.0649 |
| Mixed sample 11 | 0.0700 |
| Mixed sample 12 | 0.1000 |
| Mixed sample 13 | 0.1800 |
| Mixed sample 14 | 0.2404 |

### Example 2

220kg of each isophorone diisocyanate composition of Mixed samples 2 to 13 obtained in Example 1 and 100kg of Mobil S100 solvent oil were added into the reaction kettle, respectively. After stirring evenly, 175kg of butanone oxime was added into the reaction kettle. The reaction temperature was controlled at 60-70 °C and the reaction solution was stirred until the characteristic absorption peak of NCO could not be detected by infrared spectroscopy. This reaction process was carried out under the nitrogen protection, and finally 12 batches of blocked isophorone diisocyanate composition products were obtained for Chromatic Number testing.

600g of each blocked isocyanate composition product of the obtained 12 batches was put into a 1L three-necked flask. Under nitrogen protection, the flask was placed in an oil bath of 120 °C and the product was deblocked by heating. Through infrared monitoring, it was found that, after 2 hours, the characteristic absorption peak of NCO in all samples no longer increased. All the samples were deblocked and Chromatic Number testing was performed on the deblocked samples.

### Comparative Example 1

220kg of each of isophorone diisocyanate composition sample 1 and Mixed sample 1 obtained in Example 1 was added into a reaction kettle together with 100kg of Mobil S100 solvent oil, respectively. After stirring evenly, 175kg of butanone oxime was added into the reaction kettle. The reaction temperature was controlled at 60-70 °C and the reaction solution was stirred until the characteristic absorption peak of NCO could not be detected by infrared spectroscopy. This reaction process was carried out under nitrogen protection, and finally two batches of blocked isophorone diisocyanate composition products were obtained for Chromatic Number testing.

600g of each blocked isocyanate composition product of the obtained two batches was put into a 1L three-necked flask. Under nitrogen protection, the flask was placed in an oil bath of 120 °C and the product was deblocked by heating. Through infrared monitoring, it was found that, after 2 hours, the characteristic absorption peak of NCO in both of the samples no longer increased. Both of the samples were deblocked and Chromatic Number testing was performed on the deblocked samples.

### Comparative Example 2

220kg of each of isophorone diisocyanate composition sample 2 and Mixed sample 14 obtained in Example 1 was added into a reaction kettle together with 100kg of Mobil S100 solvent oil, respectively. After stirring evenly, 175kg of butanone oxime was added into the reaction kettle. The reaction temperature was controlled at 60-70 °C and the reaction solution was stirred until the characteristic absorption peak of NCO could not be detected by infrared spectroscopy. This reaction process was carried out under nitrogen protection, and finally two batches of blocked isophorone diisocyanate composition products were obtained for Chromatic Number testing.

600g of each blocked isocyanate composition product of the two batches obtained from sample 2 and Mixed sample 14 was put into a 1L three-necked flask. Under nitrogen protection, the flask was placed in an oil bath of 120 °C and the product was deblocked by heating. Through infrared monitoring, it was found that, after 2 hours, the characteristic absorption peak of NCO in both of the samples continued to increase, and the samples were not completely deblocked. Through infrared monitoring, it was found that, after 4 hours, the characteristic absorption peak of NCO in both of the samples no longer increased. Both of the samples were deblocked and the Chromatic Number testing was performed on the deblocked samples.

The Chromatic Number changes of the 12 batches of samples in Example 2 and the 4 batches of samples in Comparative Examples 1 and 2 during the deblocking process are shown in Table 2.

**Table 2**

| | Me-IPDI content/% | Chromatic Number of the blocked IPDI composition/hazen | | |
|---|---|---|---|---|
| | | Before deblocking | After deblocking | Chromatic Number increase |
| Sample 1 | 0.0003 | 5 | 35 | 30 |
| Mixed sample 1 | 0.0009 | 5 | 32 | 27 |
| Mixed sample 2 | 0.0029 | 6 | 21 | 15 |
| Mixed sample 3 | 0.0041 | 6 | 21 | 15 |
| Mixed sample 4 | 0.0070 | 7 | 17 | 10 |
| Mixed sample 5 | 0.0089 | 7 | 17 | 10 |
| Mixed sample 6 | 0.0201 | 9 | 12 | 3 |
| Mixed sample 7 | 0.0300 | 9 | 11 | 2 |
| Mixed sample 8 | 0.0399 | 9 | 12 | 3 |
| Mixed sample 9 | 0.0502 | 9 | 13 | 4 |
| Mixed sample 10 | 0.0649 | 12 | 22 | 10 |
| Mixed sample 11 | 0.0700 | 13 | 22 | 9 |
| Mixed sample 12 | 0.1000 | 14 | 27 | 13 |
| Mixed sample 13 | 0.1800 | 15 | 29 | 14 |
| Mixed sample 14 | 0.2404 | 17 | 39 | 22 |
| Sample 2 | 0.3200 | 18 | 43 | 25 |

The result data from Table 2 indicate that the Chromatic Number increase during the deblocking process of the 12 batches of blocked isophorone diisocyanate prepared in Example 2 is significantly lower than that of the 4 batches in the comparative examples, indicating that the stability of the blocked products prepared using the isophorone diisocyanate compositions provided in the present application is significantly superior to other products during the deblocking process.

The present application illustrates the detailed method of the present application through the above embodiments, but the present application is not limited to the above detailed methods, that is, it does not mean that the present application must rely on the above detailed method to implement. Those skilled in the relevant technical field should understand that any improvements made to the present application, equivalent substitutions of various raw materials and the addition of auxiliary components to the products of the present application, and the selection of specific methods are within the scope of protection and disclosure of the present application.

## Claims

1. An isophorone diisocyanate composition, wherein a content of methylated isophorone diisocyanate (Me-IPDI) in the isophorone diisocyanate composition is in a range from 0.002 wt% to 0.200 wt%.

2. The isophorone diisocyanate composition according to claim 1, wherein the content of Me-IPDI in the isophorone diisocyanate composition is in a range from 0.005% to 0.075%.

3. The isophorone diisocyanate composition according to claim 2, wherein the content of Me-IPDI in the isophorone diisocyanate composition is in a range from 0.010% to 0.060%.

4. The isophorone diisocyanate composition according to any one of claims 1 to 3, wherein Me-IPDI has a structural formula that is: 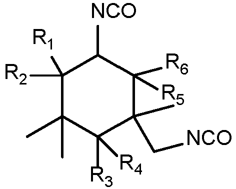 wherein at least one of R₁, R₂, R₃, R₄, R₅, and R₆ is CH₃, and the others are H.

5. The isophorone diisocyanate composition according to any one of claims 1 to 4, wherein at least one of R₁, R₃, and R₅ has a structure of CH₃.

6. The isophorone diisocyanate composition according to any one of claims 1 to 5, wherein the structure of Me-IPDI is one or more selected from the group consisting of the following structures:

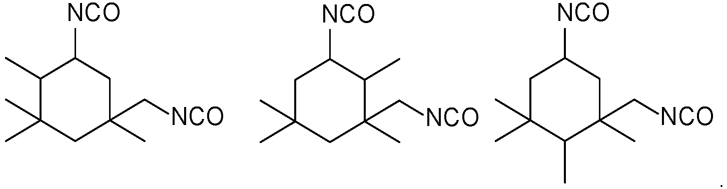

7. A method for preparing a blocked isophorone diisocyanate which is storage-stable, wherein the blocked isophorone diisocyanate is obtained by contacting the isophorone diisocyanate composition according to any one of claims 1 to 6 with a blocking agent, and performing reaction.

8. The method according to claim 7, wherein the blocking agent is one or more selected from the group consisting of oxime compounds, alcohol compounds, lactam compounds, pyrazole compounds, and *β*-dicarbonyl compounds.

9. The method according to claim 8, wherein the oxime compound is one or more selected from the group consisting of butanone oxime, acetone oxime, formaldehyde oxime, acetaldehyde oxime, and cyclohexanone oxime.

10. The method according to any one of claims 7 to 9, wherein a ratio of a molar amount of NCO in the isophorone diisocyanate composition to a molar amount of the blocking agent is (0.9-1):1.

11. The method according to any one of claims 7 to 10, wherein the reaction is carried out at a temperature ranging from 30°C to 120 °C.

12. The method according to any one of claims 7 to 11, wherein the reaction is carried out in the absence of solvent or in the presence of solvent;
Preferably, the solvent is one or more selected from the group consisting of ethyl acetate, butyl acetate, 1-methoxy-2-propyl acetate, 3-methoxy-n-butyl acetate, acetone, butanone, 4-methyl-2-pentanone, cyclohexanone, toluene, xylene, and S100 solvent oil.
